# EUROPEAN PATENT APPLICATION

(11) **EP 0 911 041 A2**
(43) Date of publication of application: **28.04.1999**
(21) Application number: 98118332.0
(22) Date of filing: 28.09.1998
(51) Int. Cl.: A61L 9/03

(54) **Device for electrically vaporizing active principles**

(30) Priority: 27.10.1997 IT BO970640
(71) Applicant: FALP S.r.l., 40060 Passo Segni Baricella (Bologna) (IT)
(72) Inventor: Lupotto, Paolo, 13020 Pila (Vercelli) (IT); Falchieri, Roberto, 40060 Passo Segni Baricella (BO) (IT)
(74) Representative: Modiano, Guido, Dr.-Ing.

(57) **Abstract**

The device for electrically vaporizing active principles comprises at least one element (3) adapted to be impregnated with a substance that contains the active principle to be vaporized and at least one heating element (1) which is adapted to produce the vaporization of the substance that contains the active principle from the impregnated element. The device has a timer which is adapted to control the flow of electric current through the heating element (1) for the selective high-temperature pulsed heating of the heating element (1) to a temperature which is close to the boiling point of the substance that contains the active principle.

## Description

The present invention relates to a device for electrically vaporizing active principles such as insecticides, deodorants, disinfectants and the like.

Conventional devices vaporize active principles of the above mentioned type in the air by producing the evaporation of a mixture which contains said active principles. This application is particularly useful in the case of insecticides, since it is possible to adjust the duration of the vaporization over a relatively long time and correspondingly control the presence of the active principle in the air.

More particularly, according to a conventional solution, the liquid that contains the active principle to be vaporized impregnates a tablet of porous material, for example made of cardboard or feltpaper, and is heated by contact with an adapted warm surface.

This method is per se simple and cheap and is substantially adapted for vaporizing single doses, for example for one night.

More recently, it has been suggested to use vaporizers which are capable of producing the evaporation of the liquid contained in a bottle provided with a wick, which is heated at the top by means of an adapted heating element. This solution is more practical in use by virtue of the longer life of the refill, which is typically equivalent, for example, to 45 nights.

The above cited solutions, however, have considerable difficulties in adjusting evaporation as required. The evaporation rate in fact depends on various factors, such as in particular the vaporization temperature, the characteristics of the liquid and those of the porous wick, besides the characteristics of the active principle vaporizing device itself.

In practice, the systems that are currently used are forced to adopt a series of technical compromises in view of the low cost, practicality and flexibility in use that vaporizer devices are required to have.

In particular, the conventional systems generally have a high level of dilution of the active principle, with a consequent waste of preparation and packaging materials, and require the use of dilution compounds which are highly volatile and therefore flammable, such as light hydrocarbons. It has also been found that it is difficult to adjust the greenhouse effect without causing irregular operation and that the active principles that can be used are limited to those which can meet the above constraints.

The aim of the present invention is to solve the cited problem, providing a device for electrically vaporizing active principles which allows to optimally adjust the evaporation of the mixture that contains the active principles, so as to allow, in particular, evaporation regardless of the chemical and physical characteristics of the substance to be vaporized.

Within the scope of this aim, an object of the present invention is to provide a device for electrically vaporizing active principles which is simple in concept, safely reliable in operation and versatile in use.

This aim, this object and others which will become apparent hereinafter, are achieved, according to the invention, by a device for electrically vaporizing active principles, comprising at least one element adapted to be impregnated with a substance that contains the active principle to be vaporized and at least one heating element which is adapted to produce the vaporization of said substance that contains the active principle from said impregnated element, characterized in that it further comprises a timer adapted to control the flow of electric current through said heating element for the selective high-temperature pulsed heating of said heating element.

The details of the invention will become apparent from the detailed description of a preferred embodiment of the device for electrically vaporizing active principles, illustrated only by way of non-limitative example in the accompanying drawings, wherein:

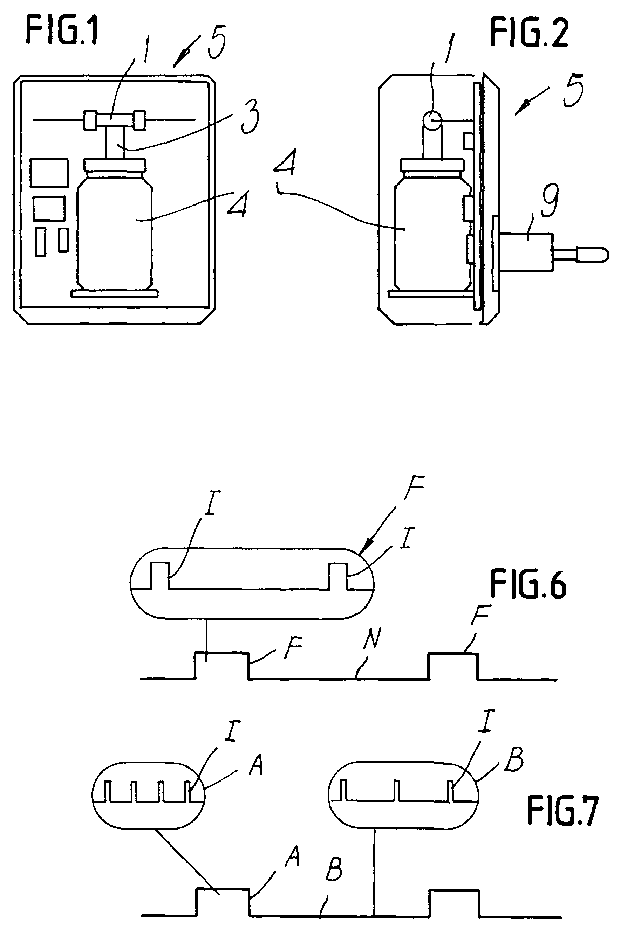

Figure 1 is a schematic front view of the device for electrically vaporizing active principles according to the invention;
Figure 2 is a corresponding side view thereof;
Figure 3 is an exploded perspective view of the device;
Figure 4 is a block diagram of the timer of the device;
Figure 5 is an electrical diagram of the timer;
Figures 6 and 7 are, respectively, diagrams of the timed operating cycles of the device in various operating conditions;
Figure 8 is a plan view of an alternative embodiment of a hood of the device;
Figure 9 is a front view of the hood of Figure 8 in the disassembled condition;
Figure 10 is an exploded perspective view of the hood of Figure 8.

With reference to the above figures, the reference numeral 1 indicates the presence of at least one heating element of the device for electrically vaporizing active principles, such as insecticides, deodorants, disinfectants and the like. The heating element 1 is preferably constituted by a small resistor with low thermal inertia, inserted in a draft hood 2 so as to generate a stream of air at high temperature which is adapted to flow over a protruding portion 3 of at least one element adapted to be impregnated with a substance containing active principles to be vaporised. The at least one element is for example a wick, the remaining portion whereof is inserted in a bottle 4 which contains a liquid mixture of the active principle to be vaporized; the wick is impregnated with said liquid.

The heating resistor can be provided with oxide layer, metallic layer, thin-film or thick-film technology on a ceramic substrate. The resistor can also be placed in contact with the liquid and the wick, provided that a path allowing to rapidly remove the generated stream of vapor is formed.

The heating element 1 is inserted in a body 5 of the device which forms an adapted seat for the bottle 4.

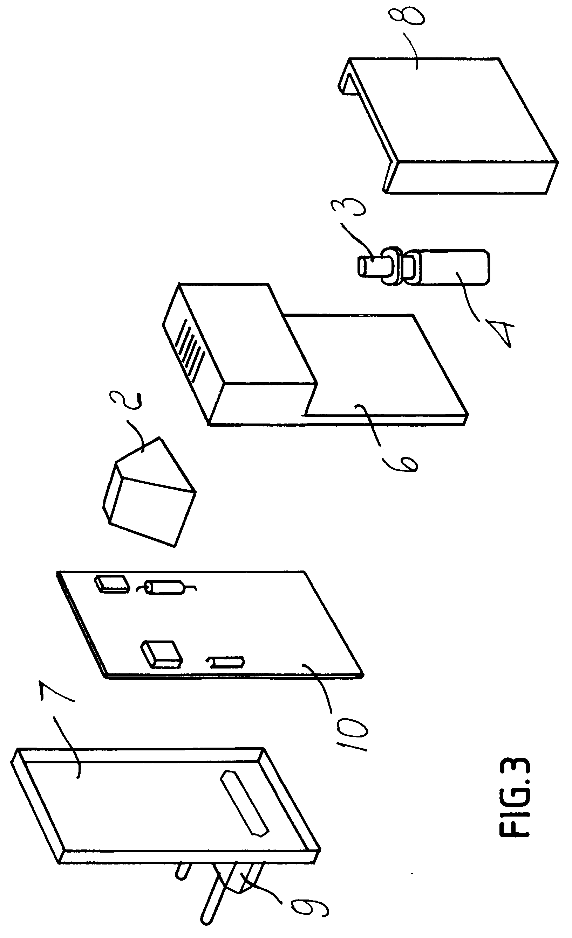

The body 5 is conveniently constituted, as shown in detail in Figure 3, by two shells 6 and 7, respectively a front shell and a rear shell, which are adapted to be mutually associated. The front shell 6 is closed by a cover 8, while the rear shell 7 is provided with a plug 9 for the supply of electric power to the device.

A base 10 is enclosed between the shells 6 and 7 in the assembly position and acts as support for an electronic timer 11 which is adapted to control the flow of electric current through the heating element 1 in order to produce selective pulsed heating at preset and adjustable times.

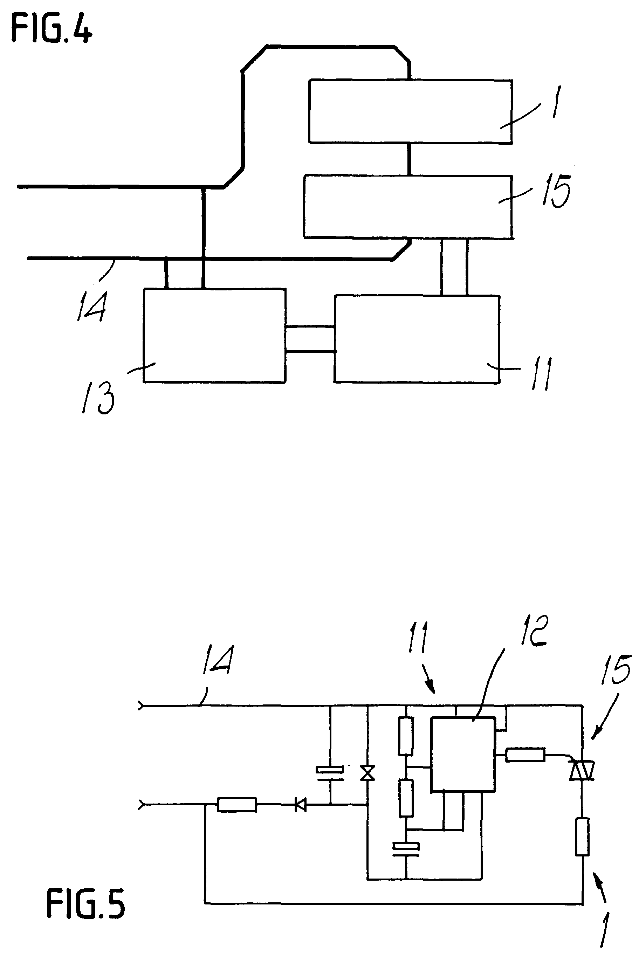

More specifically, as shown schematically in Figures 4 and 5, the timer 11 has a microprocessor 12 and is supplied by means of an adapted power supply 13 which is connected to the electric mains 14 and whose input is on the plug 9 of the device. The timer 11 controls the flow of electric current to the heating element 1 by means of a solid-state relay 15. The relay 15 detects a variation in the power supply circuit and accordingly produces a variation in the controlled circuit, i.e., in the heating element 1.

The operation of the device is easily understandable from the above description.

The timer 11 controls the flow of electric current through the heating element 1, producing consequent heating at preset and adjustable times. The electric current is emitted in pulses whose frequency and duration are appropriately adjusted by said timer 11.

In this manner, it is possible to obtain for a short time, for example in the order of 2-20 seconds, a stream at high temperature, by way of example 150-250 °C, and then leave it to cool for a significantly longer time, for example 10-400 seconds.

The resulting effect, also by virtue of the low thermal inertia of the resistor that constitutes the heating element 1, is that a high temperature, close to or higher than the boiling point of the liquid with which the wick 3 is impregnated, is applied to the wick 3 during said pulses, so as to achieve instant evaporation of the surface liquid or at least part thereof.

By varying the heating time, i.e., the duration of the pulses, the amount of liquid evaporated during each pulse is adjusted, while by varying the repetition rate of the pulses the amount of liquid vaporized during one hour is adjusted as required.

The subsequent cooling allows to keep the wick 3 at a low temperature and also allow the time required by the liquid to saturate the region of the wick that is affected by the air stream. This also helps to ensure the cleanliness of the wick 3.

If it is necessary to have shorter times or higher temperatures, the same effect can be obtained by direct contact of the heating element 1 with the surface of the wick 3.

However, depending on the nature of the liquid to be vaporized, direct contact of the wick 3 with the heating element may produce on the wick deposits which may, over time, compromise the constancy of the performance of the device, i.e., of the ratio between applied heat and amount of vaporized liquid.

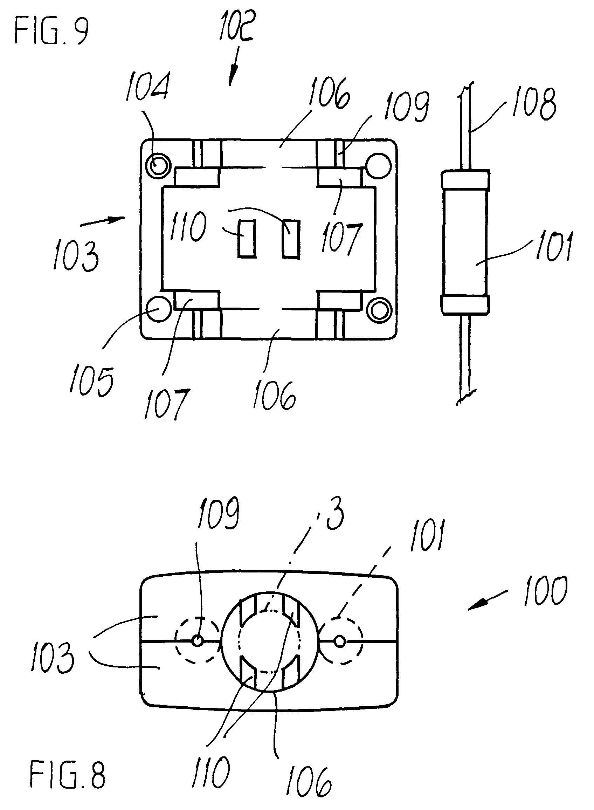

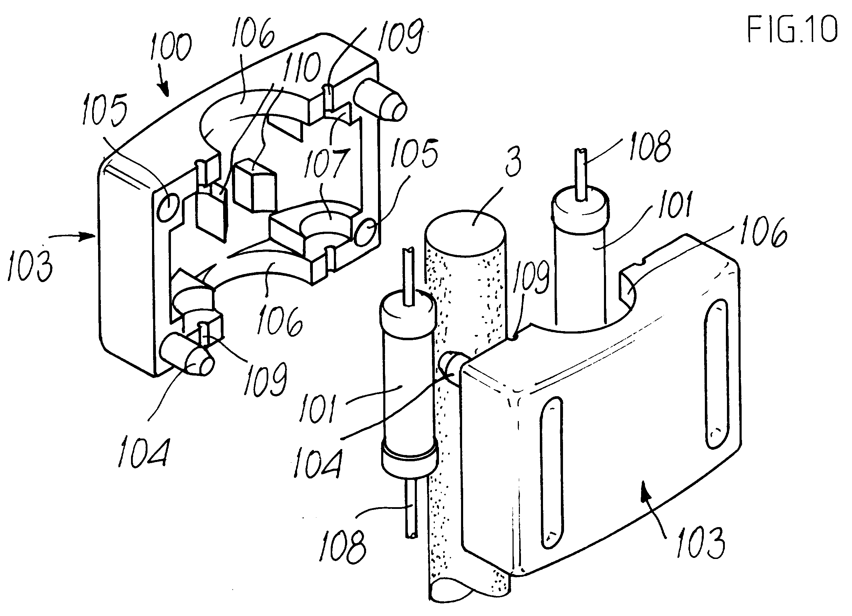

Accordingly, in a hood 100 of the device (embodiment thereof is shown in Figures 8, 9 and 10), the wick 3 is spaced in a preset manner from heating resistors 101.

The wick is meant to be inserted loosely in a hole 102 which passes through the hood 100 and remains between two half-shells 103 that constitute it.

The two half-shells 103 are mutually joined, at their edges, by means of the facing engagement of pins 104 in mutually opposite dead holes 105 which respectively protrude from, and are recessed in, the edges of both half-shells.

The hole 102 is substantially produced by the joining of two facing semicircular recesses 106 which affect the edges of the two half-shells 103 at two opposite sides of the hood 100 and have a radius which is greater than the transverse cross-section of the wick 3.

The seats of the heating resistors 101 are elongated in a direction which is parallel to the hole 102 and are open toward said hole in diametrically opposite positions. Their heads have respective seats which are formed, at said two opposite sides of the hood 100, by the coupling of two facing recesses 107 provided inside the half-shells 103.

Wires 108 for the electrical connection of the resistors 101 exit from the hood 100 through holes formed by the coupling of facing notches 109 of the edges of the half-shells 103.

The wick 3 is centered within the hole 102 and spaced from the resistors 101 by means of the contoured raised portions 110, which protrude from the front walls of the half-shells 103. Of course, it is also possible to accommodate just one resistor within the half-shells.

By means of the selective heating provided in pulses whose duration and frequency can be adjusted, the device accordingly achieves the aim of allowing a dosage which is independent of the chemical and physical characteristics of the substance to be vaporized. It is in fact always possible to reach the boiling point of the liquid and therefore achieve almost instant evaporation.

In such conditions, the amount of liquid evaporated during one pulse is proportional to the energy of the pulse and inversely proportional to the latent heat of evaporation that is characteristic of the liquid.

Furthermore, by means of the timer 11 it is possible to provide adapted cycles for modulating the emission of the active principle, such as to simulate for example the applications of an aerosol at constant times or such as to allow to eliminate insects such as flies without the risk of saturating the environment above the danger level for human beings.

Figure 6 is an exemplifying diagram of a timed operating cycle of the device of the invention, which provides for automated power-on on a daily basis to electrically vaporize insecticides or deodorants for enclosed spaces. In particular, there is for example an operating cycle F, which lasts for example 2 to 3 hours during each day. When it is not active, the device is kept inactive in the standby step N. During the operating cycle F there is a series of heating pulses I, lasting for example 0.5-2 seconds, separated by cooling periods lasting 5-60 seconds.

Figure 7 instead illustrates, again by way of example, an operating cycle of the device of the invention in which the intensity of the vaporization effect is also adjusted by modulating the frequency of the pulses.

In this case there is for example a daily operating cycle A at high intensity, lasting 2-3 hours, alternated with a step B for operation at low intensity. The high-intensity cycle A provides for a series of pulses I at intervals of 10 seconds, while the low-intensity step B provides for a series of pulses at intervals of 50 seconds.

This allows to optimize vaporization, allowing for example stronger fragrances during cooking hours, or an insecticidal effect similar to a spray.

As an alternative, it is possible to provide for the adjustment of the effect by acting only on the frequency of the heating pulses. In this case, the timer can be provided more cheaply by means of a selector with a plurality of settings or a potentiometer, without the aid of a microprocessor. The timer can of course be provided by means of any electronic or possibly mechanical technology.

Another advantage of the device according to the invention is that the amount of liquid that is present in the bottle is substantially irrelevant; accordingly, it is possible to use various degrees of dilution or pure active principles. This also allows a refill which lasts longer than current ones.

Besides evident economic advantages, this is also advantageous from the point of view of environmental impact.

The smaller volume of liquid and the possibility to use less volatile solvents also make the entire system less dangerous from the point of view of flammability, with a high self-extinguishing possibility.

The use of solutions having a high concentration of the active principle and the consequent reduced dimensions of the bottles that contain the solutions allow to install even two separate bottles even in compact devices; the impregnated elements of the bottles are meant to be subjected to respective heating elements. During operation, the timer of the device, according to the instructions given to it, selectively activates either of the two heating elements, determining which of the two active principles is to be vaporized into the environment and in what manner it is to be vaporized. Such an embodiment of the device is useful for example to vaporize a deodorant during the day and a mosquito-specific insecticide during the night.

The power source of the device can be constituted not only by the electrical mains but also by rechargeable or nonrechargeable batteries. The considerable efficiency of the thermal cycle, by virtue of the higher temperature differential, allows very low power consumptions (an average heating power of less than 0.4 W, while the power of conventional devices is 4 W).

In the practical embodiment of the invention, the materials used, as well as the shapes and the dimensions, may be any according to the requirements.

The disclosures in Italian Patent Application No. BO97A000640 from which this application claims priority are incorporated herein by reference.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. A device for electrically vaporizing active principles, comprising at least one element adapted to be impregnated with a substance that contains the active principle to be vaporized and at least one heating element which is adapted to produce the vaporization of said substance that contains the active principle from said impregnated element, characterized in that it further comprises a timer adapted to control the flow of electric current through said heating element for selective high-temperature pulsed heating of said heating element.

2. The device according to claim 1, characterized in that said timer controls the flow of electric current to said heating element by means of a solid-state relay which detects a variation in the power supply circuit of said timer and accordingly produces a variation in the heating element.

3. The device according to claim 1, characterized in that said heating pulses are adjusted in terms of frequency and duration by said timer.

4. The device according to claim 1, characterized in that said impregnated element, during said pulses, is subjected to a temperature which is close to the boiling point of said substance that contains said active principle that impregnates said element, so as to achieve instant evaporation of said substance that contains said active principle.

5. The device according to claim 1, characterized in that said impregnated element is constituted by a wick which protrudes from at least one bottle which contains said active principle in a liquid mixture.

6. The device according to claim 5, characterized in that it comprises two bottles with a corresponding wick and two respective heating elements, and in that said timer selectively activates either of said heating elements.

7. The device according to claim 5, characterized in that it comprises: a hood through which a hole passes, said wick being meant to be inserted loosely in said hood; seats which are formed in said hood so as to accommodate at least one of said heating elements and are open toward said hole; raised portions for centering said wick in said hole so that the wick is spaced from said heating element.
